# EUROPEAN PATENT SPECIFICATION

(11) **EP 4 045 117 B1**
(45) Date of publication and mention of the grant of the patent: **03.12.2025**
(21) Application number: 20876338.3
(22) Date of filing: 09.10.2020
(51) Int. Cl.: A61M 16/00, A61M 16/10, A61M 16/20

(54) **SYSTEMS OF SUPPLYING THERAPEUTIC GAS BASED ON INHALATION DURATION**
SYSTEME ZUR ZUFUHR VON THERAPEUTISCHEM GAS BASIEREND AUF DER INHALATIONSDAUER
SYSTÈMES D'ALIMENTATION EN GAZ THÉRAPEUTIQUE BASÉS SUR LA DURÉE D'INHALATION

(30) Priority: 14.10.2019 US 201962914647 P
(43) Date of publication of application: 24.08.2022
(73) Proprietor: Incoba LLC, Wildwood, Missouri 63038 (US)
(72) Inventor: AYLSWORTH, Alonzo C., Wildwood, Missouri 63038 (US); SPECTOR, Lawrence C., Wildwood, Missouri 63038 (US)
(74) Representative: Shipp, Nicholas
(86) International application number: PCT/US2020/054942
(87) International publication number: WO 2021/076412

(56) References cited:
- WO-A1-2008/077003
- US-A- 4 686 974
- US-A- 5 287 849
- US-A- 5 701 883
- US-A1- 2005 011 523
- US-A1- 2006 005 842
- US-A1- 2006 169 281
- US-A1- 2016 136 370
- US-B2- 7 013 898

## Description

### CROSS-REFERENCE TO RELATED APPLICATIONS

This application claims the benefit of U.S. Provisional Application No. 62/914,647 filed October 14, 2019 and titled "Methods and Systems of Supplying Therapeutic Oxygen,".

### BACKGROUND

Patients with respiratory ailments may be required to breathe a therapeutic gas, such as oxygen. The therapeutic gas may be delivered to the patient from a therapeutic gas source by way of a nasal cannula. In some cases, therapeutic gas is delivered to a patient in continuously. That is, in continuous delivery the therapeutic gas is supplied at a constant flow rate throughout the patient's entire breathing cycle (i.e., both inhalation and exhalation).

US 7,013,898 B2 describes a method and apparatus for supplying respiratory oxygen to a patient where the oxygen flow is monitored so as to control and conserve oxygen in response to the pressure in the nasal passages of the patient.

US 2006/0169281 A1 describes a method and system of continuous flow selective delivery, including methods comprising sensing an attribute of respiratory airflow of a first breathing orifice of a patient, and delivering a continuous flow of therapeutic gas to a second breathing orifice of the patient simultaneously with the sensing.

US 2005/0011523 A1 describes a method and system of individually controlling positive airway pressure of a patient's nares, including a method comprising applying therapeutic gas pressure within a first naris of a patient during respiration, and applying therapeutic gas pressure within a second naris of the patient during the respiration. The therapeutic gas pressures applied to each naris are different.

WO 2008/077003 A1 describes a single blower positive airway pressure device and related method, including systems comprising a first outlet port configured to couple to a first breathing orifice of a patient, a second outlet port configured to couple to a second breathing orifice of a patient, a single blower fluidly coupled to the first outlet port and the second outlet port (the single blower provides substantially all the therapeutic gas inspired through the first and second breathing orifices, and a first valve fluidly coupled between the single blower and the first outlet port). The system is configured to control pressure of therapeutic gas applied to the first outlet port to be different than pressure of therapeutic gas applied to the second outlet port.

### SUMMARY

The invention is defined by the appended claims. One example not part of the invention, defines a method of providing therapeutic, the method comprising: sensing a current inhalation; providing a flow of therapeutic gas based on the sensing; and ceasing the flow of therapeutic gas to the patient based on a value indicative of previous inhalation duration.

In the example method, ceasing the flow of therapeutic gas may further comprise ceasing based on a predetermined percentage of the value indicative of previous inhalation duration. Ceasing flow may further comprise ceasing the flow after a flow duration being sixty percent of the value indicative of previous inhalation duration. The example method may further comprise assigning the value indicative of previous inhalation duration to be a duration of an immediately previous inhalation. The example method may further comprise assigning the value indicative of previous inhalation duration to be an average duration of a plurality of previous inhalations. The example method may further comprise assigning the value indicative of previous inhalation duration to be an average duration of three previous inhalations.

The example method may further comprise, after the ceasing, pre-charging a cannula with therapeutic gas prior to a subsequent inhalation of the patient. Pre-charging the cannula may further comprise providing a volume of therapeutic to the cannula that displaces only the gas in the cannula.

The example method may further comprise determining a most open airway, and wherein providing the flow of therapeutic gas further comprises providing the flow to the most open airway.

In the example method, providing the flow of therapeutic gas may further comprise providing from a source of therapeutic gas having a dead-head pressure of 8 psig or less.

The invention provides a therapeutic gas delivery device comprising:
a controller; a first sensor electrically coupled to the controller and configured to fluidly couple to a first hose connection, the first sensor senses an attribute of airflow through the first hose connection; a first valve electrically coupled to the controller and configured to fluidly couple a source hose connection to the first hose connection; a second sensor electrically coupled to the controller and configured to fluidly couple to a second hose connection, the second sensor senses an attribute of airflow of the second hose connection; and a second valve electrically coupled to the controller and configured to fluidly couple the source hose connection to the second hose connection. The controller may be configured to: sense a current inhalation by way of the first sensor or the second sensor; provide, based on the sensing, a flow of therapeutic gas to the first hose connection by way of the first valve; and cease the flow of therapeutic gas to the first hose connection based on a value indicative of previous inhalation duration.

In the example therapeutic gas delivery device, when the controller ceases the flow of therapeutic gas, the controller may be further configured to cease based on a predetermined percentage of the value indicative of previous inhalation duration. When the controller ceases the flow of therapeutic gas, the controller may be further configured to cease the flow after a flow duration being sixty percent of the value indicative of previous inhalation duration. The controller may be further configured to assign the value indicative of previous inhalation duration to be a duration of an immediately previous inhalation. The may be further configured to assign the value indicative of previous inhalation duration to be an average duration of a plurality of previous inhalations. The controller may be further configured to assign the value indicative of previous inhalation duration to be an average duration of three previous inhalations.

In the example therapeutic gas delivery device, the controller may be further configured to, after the cessation, provide a pre-charge of therapeutic gas to both the first and second hose connections prior to a subsequent inhalation.

In the example therapeutic gas delivery device, the controller may be further configured to determine, based on the first and second sensors, that the first hose connection has a greater flow volume.

Another example system comprises: a source of therapeutic gas; a bifurcated nasal cannula; and a therapeutic gas delivery device coupled to the source of therapeutic gas, and coupled to the bifurcated nasal cannula. The therapeutic gas delivery device may comprise: a controller; a first sensor electrically coupled to the controller and configured to fluidly couple to first lumen of the bifurcated nasal cannula, the first sensor senses an attribute of airflow through the first lumen; a first valve electrically coupled to the controller and configured to fluidly couple the source of therapeutic gas to the first lumen; a second sensor electrically coupled to the controller and configured to fluidly couple to a second lumen of the bifurcated nasal cannula, the second sensor senses an attribute of airflow of the second lumen; and a second valve electrically coupled to the controller and configured to fluidly couple the source of therapeutic gas to the second lumen. The controller may be configured to: sense a current inhalation by way of the first sensor or the second sensor; and provide a flow of therapeutic gas to the first lumen of the bifurcated nasal cannula for a time duration determined based on a value indicative of previous inhalation duration.

In the example system, when the controller provides the flow of therapeutic gas, the controller may be further configured to provide the flow based on a predetermined percentage of the value indicative of previous inhalation duration. When the controller provides the flow of therapeutic gas, the controller may be further configured to provide the flow based for the time duration being sixty percent of the value indicative of previous inhalation duration.

In the example system, the controller may be further configured to assign the value indicative of previous inhalation duration to be a duration of an immediately previous inhalation. The controller may be further configured to assign the value indicative of previous inhalation duration to be an average duration of a plurality of previous inhalations. The controller may be further configured to assign the value indicative of previous inhalation duration to be an average duration of three previous inhalations.

In the example system, the controller may be further configured to, after the providing the flow, fill at least one lumen of the bifurcated nasal cannula with therapeutic gas prior to a subsequent inhalation. Each lumen of the bifurcated nasal cannula defines a lumen volume, and wherein when the controller fills the at least one lumen of the bifurcated nasal cannula, the controller may be configured to provide to the at least one lumen a fill volume that is within plus or minus ten percent (+/- 10%) of the lumen volume.

In the example system, the controller may be further configured to determine, based on the first and second sensors, that the first lumen has a greater flow volume.

In the example system, the source of therapeutic gas may be an oxygen concentrator.

### BRIEF DESCRIPTION OF THE DRAWINGS

For a detailed description of example embodiments, reference will now be made to the accompanying drawings in which:
Figure 1 shows a system in accordance with at least some embodiments;
Figure 2 shows system in accordance with at least some embodiments;
Figure 3 shows a method in accordance with at least some embodiments; and
Figure 4 shows a controller in accordance with at least some embodiments.

### DEFINITIONS

Various terms are used to refer to particular system components. Different companies may refer to a component by different names - this document does not intend to distinguish between components that differ in name but not function. In the following discussion and in the claims, the terms "including" and "comprising" are used in an openended fashion, and thus should be interpreted to mean "including, but not limited to...." Also, the term "couple" or "couples" is intended to mean either an indirect or direct connection. Thus, if a first device couples to a second device, that connection may be through a direct connection or through an indirect connection via other devices and connections.

"Nares" shall mean the nostrils of a patient.

"Naris" shall mean a single nostril of a patient, and is the singular of "nares."

"About," in reference to a recited value, shall mean the recited value plus or minus +/- ten percent (10%).

"Controller" shall mean, alone or in combination, individual circuit components, an application specific integrated circuit (ASIC), a microcontroller with controlling software, a digital signal processor (DSP), a processor with controlling software, a programmable logic device (PLD), or a field programmable gate array (FPGA), configured to read inputs and drive outputs responsive to the inputs.

### DETAILED DESCRIPTION

The following discussion is directed to various embodiments of the invention. Although one or more of these embodiments may be preferred, the embodiments disclosed should not be interpreted, or otherwise used, as limiting the scope of the disclosure, including the claims. In addition, one skilled in the art will understand that the following description has broad application, and the discussion of any embodiment is meant only to be exemplary of that embodiment, and not intended to intimate that the scope of the disclosure, including the claims, is limited to that embodiment.

Many patients are provided therapeutic gas to address respiratory ailments, such as chronic obstructive pulmonary disease (COPD), pulmonary fibrosis, or oxygen desaturation issues associated with sleep disorders (e.g., central or obstructive sleep apnea). In situations where the supply of therapeutic gas is of little or no concern (e.g., hospital), patients may be provided therapeutic gas continuously. In particular, the patient may wear a nasal cannula that has prongs or outlet ports that are associated, one each, with the nares of the patient. The therapeutic gas may flow continuously to both nares at a predetermined flow rate (e.g., 3 liters per minute (LPM)). That is, regardless of whether the patient is inhaling or exhaling the therapeutic gas continuously flows, and thus delivery by this method is referred to as "continuous flow." A similar situation may exist for non-ambulatory patients in the home environment, where the therapeutic gas is provided from an oxygen concentrator. Again, in the home environment, and regardless of whether the patient is inhaling or exhaling, the therapeutic gas continuously flows.

In other situations, however, the supply of therapeutic gas cannot be considered endless, and thus additional devices and methods may be implemented to reduce therapeutic gas consumption. For ambulatory patients, a small portable therapeutic gas cylinder may be used to provide therapeutic gas. While theoretically possible to have the therapeutic gas continuously flow when the source is a small portable gas cylinder, providing the therapeutic gas continuously makes the supply of therapeutic gas available only for a short period of time. For that reason, related-art systems include devices that conserves therapeutic gas, and the devices are thus referred to as "conservers." In particular, conservers may be associated with small portable gas cylinders, and conservers extend the time the patient may use the small portable gas cylinder by providing a short burst or bolus of therapeutic gas at the beginning of each inhalation (e.g., for the first 100 milliseconds (ms) of the inhalation). More particularly still, gas cylinders, even when low, may have several hundred pounds of pressure. The bolus of therapeutic gas delivered by a conserver thus has relatively high pressure (e.g., 20 PSIG or more) which provides a high velocity burst to help force the bolus into the lungs of the patient.

The volume of therapeutic gas delivered in the conserve mode is selected by a clinician during patient titration. In many cases, the patient is titrated with a continuous flow system, and when the patient uses a system in the conserve mode, the volume of therapeutic gas provided in each inhalation is controlled or set using a known relationship between continuous flow prescription flow rate and conserve-mode volume. In particular, in situations where the patient is titrated using continuous flow, in the conserve mode the patient may be provided 16.5 milliliters (mL) of volume for every 1 LPM of continuous flow prescription flow rate. A few points to keep in mind before proceeding. In the conserve mode the patient is provide a bolus of therapeutic gas in each inhalation. The pressure of the therapeutic gas in each bolus is relatively high, such as 20 PSIG or more, and in some cases about 25 PSIG. Moreover, the volume of the bolus of therapeutic gas provided in each inhalation is fixed based on the prescription volume. That is, if the patient is titrated on a continuous flow system, in the conserve mode the volume of each bolus is fixed by the relationship above (e.g., 16.5 mL for each 1 LPM of continuous flow prescription flow rate). If the patient is titrated on a conserver system, the patient is provided the bolus prescription volume in each inhalation. In conserver systems the volume of therapeutic gas provided does not change in relationship to the breath rate or inhalation duration. The specification now turns to considerations regarding irritation of mucosal tissue.

The nasal cavities of a human are lined with mucosal tissue that performs multiple functions. During inhalation, the mucosal tissue humidifies and warms inhaled air. During exhalation, the mucosal tissue extracts humidity from the exhaled gas in preparation for the next inhalation. In fact, the relative humidity of gasses exhaled approaches one hundred percent for normally functioning humans. The gas inhaled into the lungs and exhaled from the lungs by a human thus has high humidity. However, therapeutic gas, such as oxygen, has very low humidity. For example, oxygen produced by an oxygen concentrator has at or near zero percent relative humidity. Oxygen from other sources, such as a large gas cylinders, portable gas cylinders, or oxygen ports in hospital rooms, likewise has very low humidity (e.g., zero percent).

When the therapeutic gas has low humidity, the therapeutic gas tends to dry the mucosal tissue. Drying of the mucosal tissue may cause everything from mild discomfort to excessive bleeding. Moreover, dryness and irritation of the mucosal tissue can cause swelling, which may exacerbate several ailments (e.g., snoring and sleep apnea). The inventors of the current specification have discovered that the drying occurs not only during inhalation, but also substantially occurs during exhalation. That is, when therapeutic gas with low or no humidity flows into the nasal cavities just before and during exhalation, the therapeutic gas still causes drying of the mucosal tissue and concomitant irritation. For example, in the pause between inhalation and exhalation (e.g., at the point when inhaled gas velocity reaches zero), the therapeutic gas continues to flow into the upper airway of the patient, causing continued drying of the mucosal tissue. Moreover, during active exhalation, the therapeutic gas mixes with the exhaled gasses in the nasal cavities, reducing relative humidity and thus causing drying. Stated otherwise, the inventors of the present specification have found that continuous flow of therapeutic gas causes irritation during both inhalation and exhalation.

Related-art devices attempt to address the drying issue by including a humidifier that increases the humidity of the therapeutic gas. However, humidifiers become a cesspool of bacteria, after even a single use, leading to bacterial infections of the sinuses and the lungs. Thus, any new system or method that provides sufficient therapeutic gas, yet reduces the irritation caused by drying of the mucosal tissue, would provide a competitive advantage in the marketplace.

Various example embodiments are directed to systems and related methods that reduce drying caused by supplying therapeutic gas to the patient. More particularly, example embodiments are directed to situations where the patient is being supplied therapeutic gas from a low-pressure source, such as an oxygen concentrator, where the pressure of the source does not support bolus delivery in a conserve mode. More particularly still, example embodiments are directed to methods and related systems to reduce drying of mucosal tissue by providing continuous flow of therapeutic gas at the continuous flow prescription flow rate only during inhalation, and in further cases only for a predetermined percentage of each inhalation. The specification now turns to an example system.

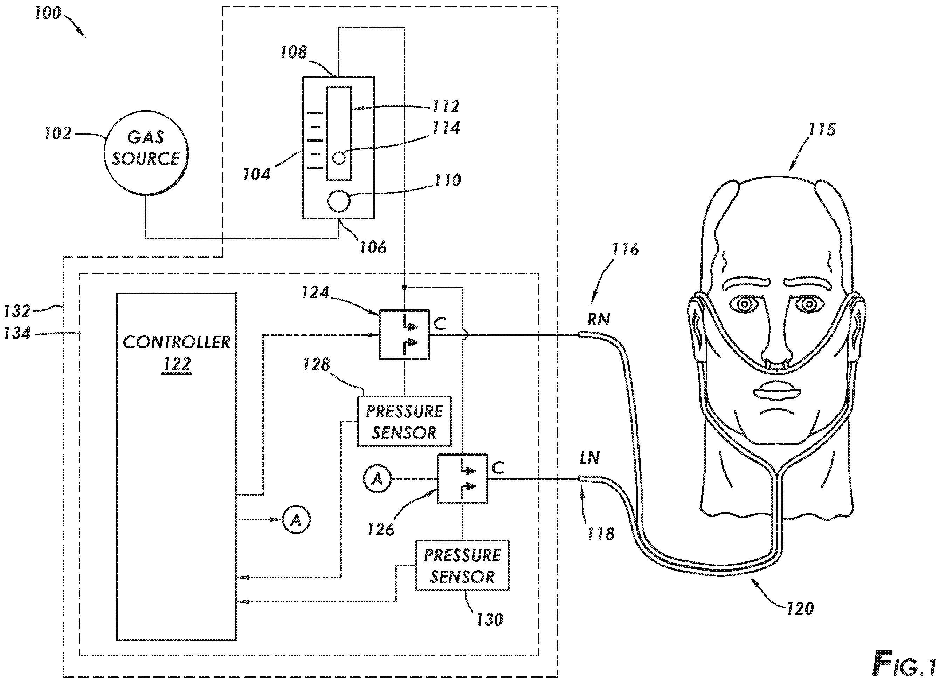

Figure 1 shows a system 100 in accordance with at least some embodiments. The example system 100 comprises a gas source 102. The gas source 102 may take any suitable form, such as an oxygen concentration system or a low-pressure permanent supply, such as in a hospital. The example gas source 102 provides or produces therapeutic gas at a relatively low pressure, in some cases 10 PSIG or less, in other cases 8 PSIG or less, and in a particular case about 5 PSIG. In order to control the flow rate of therapeutic gas, the example system comprises a flow control device in the example form of an adjustable flow meter 104. In particular, the example adjustable flow meter 104 defines an inlet port 106 at the bottom of the adjustable flow meter 104, and the inlet port 106 is fluidly coupled the gas source 102. The example adjustable flow meter 104 defines an outlet port 108 at the top of the adjustable flow meter 104, and the outlet port 108 is fluidly coupled to a delivery system (discussed more below). The example adjustable flow meter 104 further comprises a control knob 110 as well as a viewing window 112 within which a floating ball 114 is visible. The location of the floating ball 114 within the viewing window 112 is indicative of the flow rate of therapeutic gas through the adjustable flow meter 104. The rotational orientation of the control knob 110 may control the flow rate of therapeutic gas through the adjustable flow meter 104. Thus, in some cases the clinician and/or the patient adjusts the control knob 110 such that the floating ball 114 shows the flow rate to be at the continuous flow prescription flow rate (e.g., between and including 1 LPM and 6 LPM).

The example system 100 couples to a patient 115 by way of a variety of ports or hose connections, such as a right naris hose connection 116 and a left naris hose connection 118. For example, the system 100 may couple to a patient's nares by way of dual-lumen or bifurcated nasal cannula 120, and lumens of the nasal cannula couple to the hose connections 116 and 118.

In accordance with at least some embodiments, the system 100 comprises both electrical components and mechanical connections. In order to differentiate between electrical connections and mechanical connections, Figure 1 (and the remaining figures) illustrate electrical connections between components with dashed lines, and fluid connections (e.g. tubing connections between devices) with solid lines. The example system 100 of Figure 1 comprises controller 122. The example controller 122 may drive on/off or Boolean signals, such as signals to control the state of various electrically-controlled valves. Moreover, the example controller 122 may read signals (e.g., from various sensors) indicative inhalations and exhalations through the breathing orifices of the patient 115.

The example system 100 thus comprises electrically-controlled valves in the example form of three-port valve 124 and three-port valve 126. Each of these three-port valves may be a five-volt solenoid operated valve that selectively couples one of two ports to a common port (each common port labeled as C in the figure). Three-port valves 124 and 126 may be Humprey Mini-Mizers having part No. D3061, available from the John Henry Foster Co., or equivalents.

A first port or normally-open port of the three-port valve 124 fluidly couples to the adjustable flow meter 104. A second port or normally-closed port of the three-port valve 124 fluidly couples a sensor in the example form of a pressure sensor 128. The common port of the three-port valve 124 fluidly couples to the right naris hose connection 116. Similarly, a normally-open port of the three-port valve 126 fluidly couples to the adjustable flow meter 104. A normally-closed port of the three-port valve 126 fluidly couples a sensor in the example form of a pressure sensor 130. The common port of the three-port valve 126 fluidly couples to the left naris hose connection 118. The example three-port valves 124 and 126 have their normally-open ports coupled to the adjustable flow meter such that, in the absence of power, the system 100 defaults to flowing the continuous flow prescription flow rate split between the left naris and right naris.

Each three-port valve 124 and 126 is electrically coupled to the controller 122. By selectively applying voltage from the controller 122 on a respective electrical connection, the controller 122 may be able to control the flow state of the system 100. For example, with respect to the three-port valve 124, the three-port valve 124 may: couple therapeutic gas from the adjustable flow meter 104 to the common port and therefore to the example right naris; and couple the pressure sensor 128 to the common port and therefore the example right naris. Likewise, the three-port valve 126, under command of the controller 122, may: couple therapeutic gas from the adjustable flow meter 104 to the common port and therefore the example left naris; and couple the pressure sensor 130 to the common port and therefore the example left naris.

The example pressure sensors 128 and 130 are electrically coupled to the controller 122 such that the controller 122 can read the pressure and/or flow sensed by each sensor. More particularly, the controller 122 may read values indicative of inhalation airflow through each respective breathing orifice, and the controller 122 may read values indicative of exhalation airflow through each breathing orifice. In alternative embodiments, the pressure sensors 128 and 130 couple to the common ports of the three-port valves 124 and 126, respectively, if the pressure sensors can withstand the pressure of the therapeutic gas during delivery without damage. If the pressure sensors couple directly to the common port of each three-port valve, the second port of each three-port valve may be blocked. Regardless of the precise placement, the controller 122 may be able to read an indication of when the patient 115 is inhaling, read an indication of when the patient 115 is exhaling, and read an indication of how much of the air drawn by the patient 115 flows through each naris.

Still referring to Figure 1. As the patient inhales, outlet ports of the nasal cannula 120 proximate to the openings of each naris experience a pressure drop. The pressure drop may be sensed through the nasal cannula 120 and associated tubing by each of the pressure sensors 128 and 130. Likewise, as the patient exhales the outlet ports of the nasal cannula 120 proximate to the openings of each naris experience a pressure increase. The pressure increase may be sensed through the nasal cannula 120 and associated tubing by each of the pressure sensors 128 and 130. In accordance with various embodiments, the system 100 senses inhalations and exhalations, and provides therapeutic gas to the patient 115 in a current inhalation based on a duration of one or more previous inhalations.

If there is no obstruction to inhalation in either of the nares, therapeutic gas may be provided to only one naris in each inhalation. In particular, in one example case therapeutic gas is provided to the naris that has the greatest inhalation airflow. Stated otherwise, in one example case the therapeutic gas is provided to the most open airway. If the nares have or carry substantially the same inhalation airflow, the example system may alternate delivery location breath-to-breath. In other cases, the therapeutic gas may be delivered to only one naris for a plurality of inhalations, and the delivery location may alternate to the second naris for a plurality of inhalations.

The system 100 itself may take several forms. In some cases, the system 100 may be an integrated system in which the patient leases, rents, or buys the entire system. For example, when the gas source 102 is a small gas cylinder of therapeutic gas, the gas cylinder, the adjustable flow meter 104, and the remaining valves and sensors may be a single commercial or retail product. In other cases, the gas source 102 may be a standalone component coupled to an integrated system that comprises the adjustable flow meter 104 and the remaining valves and sensors, the example integrated system shown by dashed line 132. In yet still further cases, the gas source 102, the adjustable flow meter 104, and the delivery system shown by dashed line 134 (hereafter delivery system 134) may be separate components combined together to form the overall system 100.

Consider an initial situation in which the delivery system 134 has just been powered on and thus has not collected data regarding duration of prior inhalations. In the initial situation or startup mode, the delivery system 134 may sense inhalations of the patient 115. In some cases, the delivery system 134 may refrain from providing therapeutic gas for the first inhalation or the first plurality of inhalations, while simultaneously gathering data regarding inhalation duration(s). In other cases, the delivery system 134, in the absence of data regarding previous inhalation duration(s), may provide the therapeutic gas for a predetermined duration (e.g., 600 ms) in each inhalation while data regarding inhalation duration(s) are gathered. During the example startup mode, to the extent the delivery system 134 is providing therapeutic gas, the delivery system 134 only controls the duration of the continuous flow delivery (e.g., for the predetermined duration). The flow rate of therapeutic gas is controlled by the adjustable flow meter 104 set to the continuous flow prescription flow rate.

Now consider that the example delivery system 134 has sensed one or a plurality of previous inhalations, and thus has collected data regarding previous inhalation duration(s). Based on the at least one previous inhalation duration, the delivery system 134 (e.g., the controller 122) creates or assigns a value indicative of previous inhalation duration. If the value indicative of previous inhalation duration is based on a single previous inhalation (e.g., the immediately previously inhalation), then the controller 122 may assign the sensed duration of the previous inhalation to be the value indicative of previous inhalation duration. If based on a plurality of previous inhalations (e.g., previous three inhalations, inhalations in the last minute), the controller 122 may create or assign the value indicative of previous inhalation duration based the duration of the previous inhalations. For example, in some cases the controller 122 may average the previous inhalation durations to create the value indicative of previous inhalation duration. In yet still other cases, the controller 122 may select a duration from the plurality of previous inhalations (e.g., longest duration, shortest duration, mean duration, a duration from the top quartile of durations), and assign the selected duration to be the value indicative of previous inhalation duration. Regardless of the precise methodology used, the delivery system 134, and particularly the controller 122, creates the value indicative of previous inhalation duration.

Consider, as an example, a resting patient. Resting patients may have a breath rate in a range from 14 to 30 breaths per minute (BPM), with 20 BPM being a normal resting breath rate. At 20 BPM the typical breath comprises a one second inhalation, followed by a two second exhalation. Thus, for a resting patient with a 20 BPM breath rate as described, the controller 122 may assign the value indicative of previous inhalation duration to be about one second. The specification now turns to a more detailed description of operation of the example system.

In accordance with example embodiments, the delivery system 134 senses a current inhalation of the patient 115. The sensing may be by sensing an attribute of airflow through the right naris (e.g., using the pressure sensor 128), the sensing may be by sensing an attribute of airflow through the left naris (e.g., using the pressure sensor 130), or a combination of both. Triggered by the sensing of an inhalation, the example delivery system 134 provides a continuous flow of therapeutic gas to the patient 115. In some cases, the continuous flow of therapeutic gas is provided to the most open naris. If both nares carry substantially the same amount of flow, the delivery system 134 may randomly select a naris for delivery, may alternate between nares inhalation-to-inhalation, or may alternate after a predetermined number of inhalations with delivery to a particular naris. While the delivery system 134 selects the naris for delivery of the continuous flow, the flow rate of therapeutic gas is controlled by the adjustable flow meter 104 set to the continuous flow prescription flow rate.

The example system 100 continues to provide the continuous flow of therapeutic gas, with the duration of the continuous flow controlled by the value indicative of previous inhalation duration. That is, in example systems, the continuous flow of therapeutic gas ceases based on the value indicative of previous inhalation duration. In some example cases, ceasing of the flow of therapeutic gas may occur after a delivery duration being a predetermined percentage of the value indicative of previous inhalation duration. In one example case, the ceasing of the flow occurs at about sixty percent (60%) of the value indicative of previous inhalation duration. The example sixty percent value is derived from or based on the concept of alveolar pressure. That is, during inhalation a patient's diagraph contracts which causes reduced pressure in the lungs, and the reduced pressure causes airflow into the lungs. Airflow in the early portion of the inhalation makes its way to the alveolar where gas exchange takes place. Airflow later in the inhalation may be drawn in through the nose or mouth, but may not proceed far enough into the lungs to reach the alveolar. The example sixty percent of the inhalation duration is selected to ensure that therapeutic gas is provided during periods of time when inhaled gas will reach the alveolar, but that the flow of therapeutic gas ceases prior to the end of the inhalation because the therapeutic gas inhaled after the example sixty percent point will not reach the alveolar. Ceasing the flow of therapeutic gas prior to the end of the inhalation may reduce the irritation caused by use of therapeutic gas with very low or no humidity. Other predetermined percentages are possible (e.g., 50%, or 70%), but sixty percent represents a selection based on prior studies directed to alveolar pressure.

Now consider that the patient 115 begins to move around, such as walking. For active patients the breath rate may range from 30 to 40 BPM or more. At 30 BPM the typical breath comprises a 1000 ms inhalation followed by 1000 ms exhalation. Thus, for an active patient the controller 122 may create the value indicative of previous inhalation duration to be about 1000 ms. Assuming a predetermined percentage of about sixty percent, in the next inhalation the controller 122 may deliver a continuous flow of therapeutic gas for about 600 ms at the continuous flow prescription flow rate. That is to say, the delivery time as between the resting case and the active case is shorter for the active case, with the change in delivery duration based on the duration of one or more previous inhalations. If the continuous flow prescription flow rate is not adjusted, the total volume delivered in each inhalation will be lower. However, in some cases a clinician titrating a patient will provide multiple continuous flow prescription flow rates (e.g., one continuous flow prescription flow rate for resting, and another, higher continuous flow prescription flow rate for use during activity). Thus, in some cases the patient 115 may increase the continuous flow set point using the adjustable flow meter 104 prior to beginning or just after activity begins.

Nevertheless, adjusting the delivery duration based on the duration of one or more previous inhalations reduces excess flow of therapeutic gas (e.g., at the end of the inhalation, and during exhalation) to reduce the drying and thus reduce the irritation to the mucosal tissue of the patient. The adjustment is completed by the controller 122 without input or direction from an external source (e.g., such as a home care nurse). Thus, a COPD or pulmonary fibrosis patient may need fewer in-home visitations by a home-care nurse related to therapeutic gas adjustments, thus reducing the cost of care.

Consider, as another example, the idea of inhalation duration to exhalation duration ratio. For resting patients with early stage COPD or pulmonary fibrosis, the inhalation to exhalation relationship may be 1:2 (i.e., one second inhalation, two second exhalation), with an average breath duration being about three seconds. However, as the disease progresses, the resting breath rate increases, and correspondingly the inhalation and exhalation relationship changes. For example, as the disease progress the resting breath rate increases (e.g., one breath per second), and the inhalation to exhalation relationship approaches 1:1 (e.g., 500 ms inhalation, and 500 ms exhalation). Thus, as the patient's disease progresses, the example system 100, and particularly the controller 122, automatically adjusts the delivery duration to ensure therapeutic gas is delivered during appropriate periods to provide therapeutic gas to the alveolar, yet refraining from providing during other periods (e.g., at the end of the inhalation, and during exhalation) to reduce the drying and thus reduce the irritation to the mucosal tissue of the patient.

In the example cases discussed to the point, the ceasing of the continuous flow of therapeutic gas is based on the value indicative of previous inhalation duration. In a specific example case, the ceasing of the continuous therapeutic gas flow is a predetermined percentage of the value of indicative of previous inhalation duration. However, in other cases the system 100, and particularly the controller 122, may incorporate the predetermined percentage directly into the value indicative of previous inhalation duration. For example, the controller 122 may measure the duration of one or more previous inhalations, and determine an intermediate value using any of the examples discussed above (e.g., single inhalation duration, average of a plurality of inhalation durations). Thereafter, the controller 122 may apply the predetermined percentage to the intermediate value such that the value indicative of previous inhalation duration directly indicates the duration of providing the continuous flow of therapeutic gas in the current inhalation. In the specific example then, ceasing of the continuous flow of therapeutic gas occurs when delivery duration in the current inhalation reaches the value indicative of previous inhalation duration.

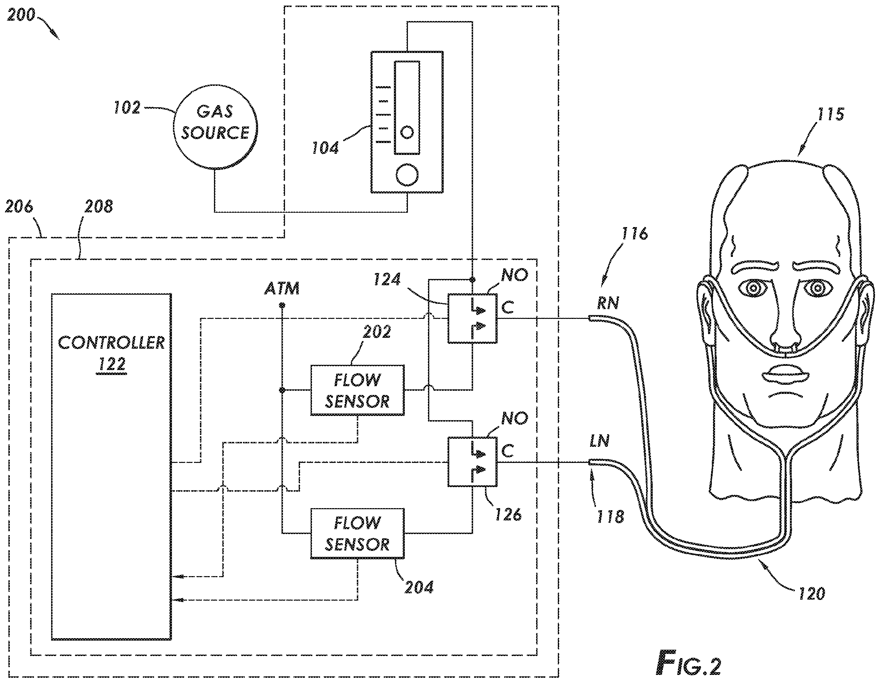

The system 100 of Figure 1 is an example system using pressure sensors to sense attributes of airflow. However, other sensors to sense attributes of airflow are possible, including flow sensors. Figure 2 shows a system 200 in accordance with at least some embodiments. Many of the components of the example system 200 are duplicated from the system 100, and thus like components share reference numbers so as not to further complicate the specification. In particular, the example system 200 comprises the gas source 102 and the adjustable flow meter 104. As before, the example system 200 couples to the patient 115 by way of a variety of ports or hose connections, such as a right naris hose connection 116 and a left naris hose connection 118. The system 200 may couple to the patient's nares by way of the bifurcated nasal cannula 120, and lumens of the bifurcated nasal cannula 120 couple to the hose connections 116 and 118. The example system 200 further comprises controller 122 to drive on/off or Boolean signals, and to read signals (e.g., from various sensors) indicative inhalations and exhalations through the breathing orifices.

As before, the example system 200 thus comprises three-port valve 124 and three-port valve 126, such as the five-volt solenoid operated valves discussed above. The normally-open port of the three-port valve 124 fluidly couples to the adjustable flow meter 104, and the normally-closed port of the three-port valve 124 fluidly couples a sensor in the example form of a flow sensor 202. The common port of the three-port valve 124 fluidly couples to the right naris hose connection 116. Similarly, the normally-open port of the three-port valve 126 fluidly couples to the adjustable flow meter 104, and the normally-closed port of the three-port valve 126 fluidly couples a sensor in the example form of a flow sensor 204. The common port of the three-port valve 126 fluidly couples to the left naris hose connection 118. Here too, the three-port valves are fluidly coupled to the gas source 102 such that, in the absence of power, the example system 200 defaults to flowing the continuous flow prescription flow rate split between the left naris and right naris. Each three-port valve 124 and 126 is electrically coupled to the controller 122.

By selectively applying voltage from the controller 122 on a respective electrical connection, the controller 122 may be able to control the state of the system 200. For example, with respect to the three-port valve 124, the three-port valve 124 may: couple therapeutic gas from the adjustable flow meter 104 to the common port and therefore to the example right naris; and couple the flow sensor 202 to the common port and therefore the example right naris. Likewise, the three-port valve 126, under command of the controller 122, may: couple therapeutic gas from the adjustable flow meter 104 to the common port and therefore the example left naris; and couple the flow sensor 204 to the common port and therefore the example left naris.

The flow sensors 202 and 204 are electrically coupled to the controller 122 such that the controller 122 can read the flow sensed by each sensor. More particularly, the controller 122 may read values indicative of inhalation airflow through each naris, and the controller 122 may read values indicative of exhalation airflow through each naris. In alternative embodiments, the flow sensors 202 and 204 couple to the common ports of the three-port valves 124 and 126, respectively, if the flow sensors can withstand the pressure of the therapeutic gas during delivery without damage. If the flow sensors couple directly to the common port of each three-port valve, the second port of each three-port valve may be blocked. Regardless of the precise placement, the controller 122 may be able to determine: an indication of when the patient 115 is inhaling; an indication of when the patient 115 is exhaling; and an indication of how much of the air drawn by the patient 115 flows through each naris.

Still referring to Figure 2. Again as the patient 115 inhales, outlet ports of the bifurcated nasal cannula 120 proximate to the openings of each naris experience a pressure drop and therefore an air flow. The air flow may be sensed through the bifurcated nasal cannula 120 and associated tubing by airflow moving through each of the flow sensors 202 and 204. That is, during inhalation the pressure drop may pull atmospheric air from the atmospheric vent (labeled ATM in the figure) through the flow sensors 202 and 204. During exhalation the increased pressure may move exhalation gas through the flow sensors 202 and 204 and out the atmospheric vent. As before, the system 200 senses inhalations and exhalations, and provides therapeutic gas to the patient 115 in a current inhalation based a value indicative of previous inhalation duration.

Operation of the example system 200, as it relates to sensing inhalation and providing the continuous flow of therapeutic gas, operates the same as discussed above with respect to Figure 1. That is, the delivery duration during a current inhalation is based on a value indicative of previous inhalation duration. Further as before, the flow rate is controlled by the adjustable flow meter 104.

As with the system 100 of Figure 1, the system 200 of Figure 2 itself may take several forms. In some cases, the system 200 may be an integrated system in which the patient leases, rents, or buys the entire system. In other cases, the gas source 102 may be coupled to an integrated system that comprises the adjustable flow meter 104 and the remaining valves and sensors, as shown by dashed line 206. In yet still further cases, the gas source 102, the adjustable flow meter 104, and the delivery system shown by dashed line 208 may be separate components combined together to form the overall system 100.

The example embodiments of Figure 2 have a potential shortcoming based exhalation gas flow into the bifurcated nasal cannula 120. That is, when using a sensor in the form of pressure sensor as in Figure 1, the volume of exhalation gas that flows into the bifurcated nasal cannula 120 is limited because gas does not flow through the pressure sensors. By contrast, when using a sensor in the form of a flow sensor as in Figure 2, the gases exhaled by the patient 115 flow into the lumens of the bifurcated nasal cannula 120. In some cases, exhaled gases may fully displace therapeutic gas remaining in the bifurcated nasal cannula 120, and depending on the length of the tubing of the bifurcated nasal cannula 120 may flow through the flow sensors 202 and 204 themselves. During the next inhalation, and considering delivery to a single naris, a volume of exhaled gas equal to the volume of the lumen of the bifurcated nasal cannula 120 is provided to the patient before therapeutic gas makes its way through the lumen and reaches the naris. As discussed above, inspired volume early in the inhalation is more likely to reach the alveolar, and thus the initial delivery of exhaled gas represents a missed opportunity.

In order to address the potential shortcomings in the case of systems using flow sensors, the example system 200 is designed and constructed to pre-charge or fill one or both of the lumens of the bifurcated nasal cannula 120 with therapeutic gas prior to a subsequent inhalation. More particularly still, prior to a next inhalation the delivery system 208 pre-charges or fills at least one lumen of the bifurcated nasal cannula 120 with therapeutic gas to displace the exhaled gas within the lumen. In some cases, the delivery system 208, and particularly the controller 122, opens an appropriate three-port valve for a period of time to fill the lumen of the bifurcated nasal cannula 120 with therapeutic gas. More particularly still, in some cases the controller 122 fills the at least one lumen (e.g., the lumen expected to be used during the next inhalation) with a volume of therapeutic gas that is within ten percent (+/- 10%) of the lumen volume. Thus, when the next inhalation is sensed, the delivery system 208 changes valve position of an appropriate three-port valve 124 or 126, and therapeutic gas almost immediately exits the ports of the bifurcated nasal cannula 120 positioned at the nares.

Consider, as an example, that the patient 115 has both nares open to flow. In a first inhalation, the inhalation is sensed by both flow sensors 202 and 204. Further consider in this example that the delivery system 208 selects the right naris as the delivery location. Thus, responsive to sensing the inhalation, the controller 122 commands the three-port valve 124 to change valve positions, and thus therapeutic gas flows from the gas source 102, though the adjustable flow meter 104, through the delivery system 208, and to the right naris of the patient 115. In conformance with various example embodiments, the delivery of therapeutic gas ceases prior to the end of the inhalation, and thus in the example situation the controller 122 commands the three-port valve 124 to again change valve positions, coupling the flow sensor 202 to the right naris. Because the patient 115 is still inhaling in this example, at least some atmospheric air is drawn through the atmospheric vent ATM, through the flow sensor 202, and into the lumen of the bifurcated nasal cannula 120 associated with the right naris. As for the left naris, during the entire inhalation atmospheric air is drawn through the atmospheric vent ATM, through the flow sensor 204, and into the lumen of the bifurcated nasal cannula 120 associated with the left naris.

At some point thereafter, the inhalation ends, and then exhalation begins. During exhalation, exhalation gas flow out of the patient's nares and into lumens of the bifurcated nasal cannula 120. In some cases the exhaled gases may fully displace the therapeutic gas remaining in the lumen associated with the right naris.

Still considering the example that the delivery system 208 intends use the right naris as the delivery location, prior to the next inhalation the delivery system 208, and particularly the controller 122, pre-charges or fills with therapeutic gas the lumen associated with the right naris. For example, in the waning stages of the subsequent exhalation, or in period between the exhalation and the next inhalation, the controller 122 may command the three-port valve 124 to change valve position to enable the therapeutic gas to flow into the lumen associated with the right naris for a period of time sufficient to displace the exhaled gas from the lumen. Inasmuch as the example systems are designed to reduce drying and thus irritation caused by use of therapeutic gas, the volume of therapeutic gas supplied in the pre-charge or fill is limited to the volume of the lumen, with a preference for under-filling rather than overfilling. When the next inhalation is sensed, again the controller 122 commands the three-port valve 124 to change valve position to enable the therapeutic gas to flow during the inhalation.

In the pre-charging above, the right naris is the selected delivery location. However, in other case the left naris is the selected delivery location, and in that case the same pre-charge of fill procedure may be used to pre-charge to the lumen associated with the left naris, and no pre-charge or filling of the lumen associated with the right naris.

Because of nasal cycle, the delivery location may change from time to time. However, rarely will a naris go from being the most open to flow to zero flow in an immediately subsequent inhalation. Thus, the example delivery systems 100 and 200 may sense, over a plurality of inhalations, that nasal resistance of a particular naris is increasing over time, and thus the delivery system 108/208 may switch selected delivery location, and likewise switch the lumen that is pre-charged, based on which naris has (or is expected to have) the greater flow volume during inhalation.

In accordance example embodiments, when the example systems 100 and 200 are powered off or in an off mode, the therapeutic gas is provided to the hose connections, and thus the nares, during both the inhalation and exhalation periods. In a second mode of operation, referred to herein as a selective delivery mode, the example systems 100 and 200 provide a continuous flow of oxygen to the most open airway of the patient 115. For example, if the right naris is the most open airway (e.g., has the lowest resistance to airflow), the continuous flow of therapeutic gas is provided only to the right naris, and the continuous flow is provided during both the both the inhalation and exhalation periods. If the most open airway changes, the monitoring and controller 122 changes the airway to which the continuous flow is provided.

In a third mode of operation, the continuous flow is provided only during the inhalation (or predetermined percentage thereof) as discussed at length above, with flow rate set by the adjustable flow meter 104. The third mode may be referred to as a pulsed liter flow.

The various embodiment discussed to this point have assumed a patient breathing at a particular rate. However, a certain portion of the population experiences sleep apnea in which the sleeper fails to breath for extended period of time (e.g., 10 seconds) and has a corresponding drop in oxygen saturation (e.g., a drop of 3% or more). In accordance with yet still other embodiments, the example systems 100 and 200 may take action when a sleep apnea is likely to occur. In particular, in example embodiments the controller 122 may detect that the next inhalation has not has not occurred within a predetermined period of time (e.g., eight seconds) after the last exhalation ended. In these situations, the example systems flow therapeutic gas to one or nares in spite of the fact that an inhalation has not started. Thus, when the patient has the breakthrough breath, the breathing airways are flooded with oxygen such that the initial volumes of the breakthrough inhalation are all or substantially oxygen. While the technique may increase drying, the technique may reduce oxygen desaturation of the patient.

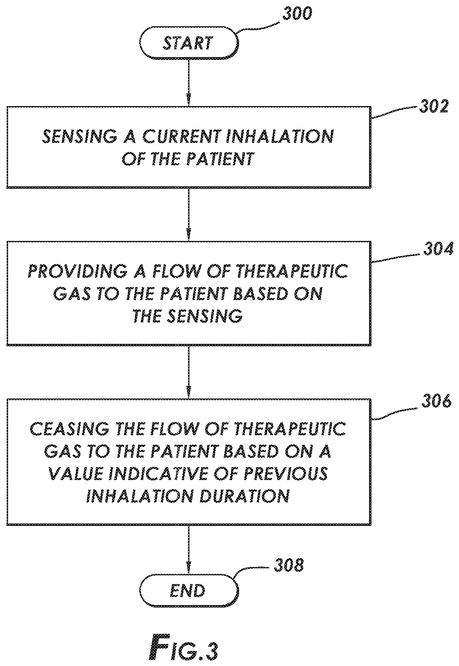

Figure 3 shows a method, which may be partially implemented in software, in accordance with at least some embodiments. In particular, the method starts (block 300) and comprises: sensing a current inhalation of the patient (block 302); providing a flow of therapeutic gas to the patient based on the sensing (block 304); and ceasing the flow of therapeutic gas to the patient based on a value indicative of previous inhalation duration (block 306). Thereafter, the method may end (block 308) likely to be restarted on the next inhalation.

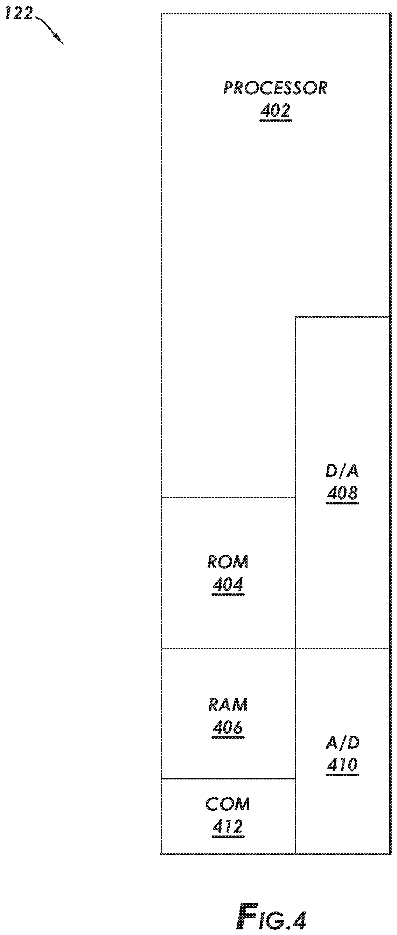

Figure 4 shows a controller 122 in accordance with at least some embodiments. The example controller 122 may be microcontroller, and therefore the microcontroller may be integral a processor 402, read only memory (ROM) 404, random access memory (RAM) 406, a digital-to-analog converter (D/A) 408, and an analog-to-digital converter (A/D) 410. The controller 122 may further comprise communication logic 412, which enables systems to communicate with external devices, e.g., to transfer stored data about a patient's breathing patterns. Although a microcontroller may be preferred because of the integrated components, in alternative embodiments the controller 122 may be implemented by a stand-alone processor 402 in combination with individual RAM, ROM, communication, D/A and A/D devices.

The ROM 404 stores instructions executable by the processor 402. In particular, the ROM 404 may comprise a software program or instructions that, in whole or in part, implements the various embodiments discussed herein. The RAM 406 may be the working memory for the processor 402, where data may be temporarily stored and from which instructions may be executed. Processor 402 may couple to other devices within the delivery system by way of A/D converter 410 (e.g., sensors to sense attributes of airflow) and D/A converter 408 (e.g., electrically-controlled valves). Thus, the ROM 404, and/or the RAM 406 may be non-transitory computer-readable mediums upon which instructions are stored.

The above discussion is meant to be illustrative of the principles and various embodiments of the present invention. Numerous variations and modifications will become apparent to those skilled in the art once the above disclosure is fully appreciated. For example, by being able to detect inhalations and exhalations, and respective durations, the system systems may be designed and constructed to determine an inhalation to exhalation relationship (e.g., 1:2 for normal breathing; or 1:1 and short breath rate for advanced COPD or pulmonary fibrosis). It is intended that the following claims be interpreted to embrace all such variations and modifications.

## Claims

1. A therapeutic gas delivery device (134) comprising:
a controller (122);
a first sensor (128) electrically coupled to the controller and configured to fluidly couple to a first hose connection, the first sensor senses an attribute of airflow through the first hose connection;
a first valve (124) electrically coupled to the controller and configured to fluidly couple a source hose connection to the first hose connection;
a second sensor (130) electrically coupled to the controller and configured to fluidly couple to a second hose connection, the second sensor senses an attribute of airflow of the second hose connection;
a second valve (126) electrically coupled to the controller and configured to fluidly couple the source hose connection to the second hose connection;
**characterized in that** the controller (122) is configured to:
sense a current inhalation by way of the first sensor or the second sensor;
provide, based on the sensing, a flow of therapeutic gas to the first hose connection by way of the first valve; and
cease the flow of therapeutic gas to the first hose connection based on a value indicative of previous inhalation duration.

2. The therapeutic gas delivery device of claim 1 wherein when the controller ceases the flow of therapeutic gas, the controller is further configured to cease based on a predetermined percentage of the value indicative of previous inhalation duration.

3. The therapeutic gas delivery device of claim 2 wherein when the controller ceases the flow of therapeutic gas, the controller is further configured to cease the flow after a flow duration being sixty percent of the value indicative of previous inhalation duration.

4. The therapeutic gas delivery device of claim 2 wherein the controller is further configured to assign the value indicative of previous inhalation duration to be a duration of an immediately previous inhalation.

5. The therapeutic gas delivery device of claim 2 wherein the controller is further configured to assign the value indicative of previous inhalation duration to be an average duration of a plurality of previous inhalations.

6. The therapeutic gas delivery device of claim 2 wherein the controller is further configured to assign the value indicative of previous inhalation duration to be an average duration of three previous inhalations.

7. The therapeutic gas delivery device of claim 1 wherein the controller is further configured to, after the cessation, provide a pre-charge of therapeutic gas to both the first and second hose connections prior to a subsequent inhalation.

8. The therapeutic gas delivery device of claim 1 wherein the controller is further configured to, after the cessation, provide a pre-charge of therapeutic gas to at least one of the first and second hose connections prior to a subsequent inhalation.

9. The therapeutic gas delivery device of claim 8, wherein each one of the first and second hose connections defines a lumen volume, and wherein when the controller fills the at least one of the first and second hose connections, the controller is configured to provide to the at least one of the first and second hose connections a fill volume that is within plus or minus ten percent (+/- 10%) of the lumen volume.

10. The therapeutic gas delivery device of claim 1 wherein the controller is further configured to determine, based on the first and second sensors, that a greater flow volume flows through the first hose connection.

11. A system comprising:
a source of therapeutic gas;
a bifurcated nasal cannula;
a therapeutic gas delivery device according to any preceding claim, where the therapeutic gas delivery device is coupled to the source of therapeutic gas, and coupled to the bifurcated nasal cannula.

12. The system of claim 11 wherein the source of therapeutic gas is an oxygen concentrator.

## Patentansprüche

1. Vorrichtung zur Zufuhr von therapeutischem Gas (134), umfassend:
eine Steuerung (122);
einen ersten Sensor (128), der elektrisch mit der Steuerung gekoppelt und dazu ausgelegt ist, fluidisch mit einer ersten Schlauchverbindung zu koppeln, wobei der erste Sensor ein Merkmal des Luftstroms durch die erste Schlauchverbindung erfasst;
ein erstes Ventil (124), das elektrisch mit der Steuerung gekoppelt und dazu ausgelegt ist, eine Quellenschlauchverbindung fluidisch mit der ersten Schlauchverbindung zu koppeln;
einen zweiten Sensor (130), der elektrisch mit der Steuerung gekoppelt und dazu ausgelegt ist, fluidisch mit einer zweiten Schlauchverbindung zu koppeln, wobei der zweite Sensor ein Merkmal des Luftstroms durch die zweite Schlauchverbindung erfasst;
ein zweites Ventil (126), das elektrisch mit der Steuerung gekoppelt und dazu ausgelegt ist, die Quellenschlauchverbindung fluidisch mit der zweiten Schlauchverbindung zu koppeln;
**dadurch gekennzeichnet, dass** die Steuerung (122) dazu ausgelegt ist:
eine aktuelle Inhalation mit dem ersten Sensor oder dem zweiten Sensor zu erfassen;
basierend auf der Erfassung einen Strom von therapeutischem Gas mithilfe des ersten Ventils an die erste Schlauchverbindung zu liefern; und
den Strom von therapeutischem Gas an die erste Schlauchverbindung basierend auf einem Wert, der eine vorherige Inhalationsdauer anzeigt, zu beenden.

2. Vorrichtung zur Zufuhr von therapeutischem Gas nach Anspruch 1, wobei, wenn die Steuerung den Strom von therapeutischem Gas beendet, die Steuerung ferner dazu ausgelegt ist, basierend auf einem vorbestimmten Prozentsatz des Wertes, der die vorherige Inhalationsdauer anzeigt, zu beenden.

3. Vorrichtung zur Zufuhr von therapeutischem Gas nach Anspruch 2, wobei, wenn die Steuerung den Strom von therapeutischem Gas beendet, die Steuerung ferner dazu ausgelegt ist, den Strom nach einer Dauer von sechzig Prozent des Wertes, der die vorherige Inhalationsdauer anzeigt, zu beenden.

4. Vorrichtung zur Zufuhr von therapeutischem Gas nach Anspruch 2, wobei die Steuerung ferner dazu ausgelegt ist, dem Wert, der die vorherige Inhalationsdauer anzeigt, eine Dauer einer unmittelbar vorherigen Inhalation zuzuweisen.

5. Vorrichtung zur Zufuhr von therapeutischem Gas nach Anspruch 2, wobei die Steuerung ferner dazu ausgelegt ist, dem Wert, der die vorherige Inhalationsdauer anzeigt, eine durchschnittliche Dauer einer Vielzahl von vorherigen Inhalationen zuzuweisen.

6. Vorrichtung zur Zufuhr von therapeutischem Gas nach Anspruch 2, wobei die Steuerung ferner dazu ausgelegt ist, dem Wert, der die vorherige Inhalationsdauer anzeigt, eine durchschnittliche Dauer von drei vorherigen Inhalationen zuzuweisen.

7. Vorrichtung zur Zufuhr von therapeutischem Gas nach Anspruch 1, wobei die Steuerung ferner dazu ausgelegt ist, nach der Beendigung, eine Vorfüllung von therapeutischem Gas vor einer nächsten Inhalation an sowohl die erste als auch die zweite Schlauchverbindung zu liefern.

8. Vorrichtung zur Zufuhr von therapeutischem Gas nach Anspruch 1, wobei die Steuerung ferner dazu ausgelegt ist, nach der Beendigung, eine Vorfüllung von therapeutischem Gas vor einer nächsten Inhalation an mindestens eine der ersten und der zweiten Schlauchverbindung zu liefern.

9. Vorrichtung zur Zufuhr von therapeutischem Gas nach Anspruch 8, wobei jede der ersten und der zweiten Schlauchverbindung ein Lumenvolumen definiert und wobei, wenn die Steuerung die mindestens eine der ersten und der zweiten Schlauchverbindung füllt, die Steuerung dazu ausgelegt ist, der mindestens einen der ersten und der zweiten Schlauchverbindung ein Füllvolumen bereitzustellen, das innerhalb von plus oder minus zehn Prozent (+/- 10%) des Lumenvolumens liegt.

10. Vorrichtung zur Zufuhr von therapeutischem Gas nach Anspruch 1, wobei die Steuerung ferner dazu ausgelegt ist, basierend auf dem ersten und dem zweiten Sensor zu bestimmen, dass ein größerer Volumenstrom durch die erste Schlauchverbindung strömt.

11. System, umfassend:
eine Quelle von therapeutischem Gas;
eine verzweigte Nasenkanüle;
eine Vorrichtung zur Zufuhr von therapeutischem Gas nach einem vorherigen Anspruch, wobei die Vorrichtung zur Zufuhr von therapeutischem Gas mit der Quelle von therapeutischem Gas gekoppelt ist und mit der verzweigten Nasenkanüle gekoppelt ist.

12. System nach Anspruch 11, wobei die Quelle von therapeutischem Gas ein Sauerstoffkonzentrator ist.

## Revendications

1. Dispositif d'administration de gaz thérapeutique (134) comprenant :
un dispositif de commande (122) ;
un premier capteur (128) couplé électriquement au dispositif de commande et configuré pour s'accoupler fluidiquement à un premier raccord de tuyau, le premier capteur détectant un attribut de flux d'air à travers le premier raccord de tuyau ;
une première valve (124) couplée électriquement au dispositif de commande et configurée pour accoupler fluidiquement un raccord de tuyau source au premier raccord de tuyau ;
un second capteur (130) couplé électriquement au dispositif de commande et configuré pour s'accoupler fluidiquement à un second raccord de tuyau, le second capteur détectant un attribut de flux d'air du second raccord de tuyau ;
une seconde valve (126) couplée électriquement au dispositif de commande et configurée pour accoupler fluidiquement le raccord de tuyau source au second raccord de tuyau ;
**caractérisé en ce que** le dispositif de commande (122) est configuré pour :
détecter une inspiration courante au moyen du premier capteur ou du second capteur ;
fournir, sur la base de la détection, un flux de gaz thérapeutique au premier raccord de tuyau au moyen de la première valve ; et
arrêter le flux de gaz thérapeutique vers le premier raccord de tuyau sur la base d'une valeur indiquant la durée d'inhalation précédente.

2. Dispositif d'administration de gaz thérapeutique selon la revendication 1 dans lequel, lorsque le dispositif de commande arrête le flux de gaz thérapeutique, le dispositif de commande est en outre configuré pour arrêter sur la base d'un pourcentage prédéterminé de la valeur indiquant la durée d'inhalation précédente.

3. Dispositif d'administration de gaz thérapeutique selon la revendication 2 dans lequel, lorsque le dispositif de commande arrête le flux de gaz thérapeutique, le dispositif de commande est en outre configuré pour arrêter le flux après une durée de flux égale à soixante pour cent de la valeur indiquant la durée d'inhalation précédente.

4. Dispositif d'administration de gaz thérapeutique selon la revendication 2 dans lequel le dispositif de commande est en outre configuré pour attribuer la valeur indiquant la durée d'inhalation précédente pour qu'elle soit une durée d'une inhalation immédiatement précédente.

5. Dispositif d'administration de gaz thérapeutique selon la revendication 2 dans lequel le dispositif de commande est en outre configuré pour attribuer la valeur indiquant la durée d'inhalation précédente pour qu'elle soit une durée moyenne d'une pluralité d'inhalations précédentes.

6. Dispositif d'administration de gaz thérapeutique selon la revendication 2 dans lequel le dispositif de commande est en outre configuré pour attribuer la valeur indiquant la durée d'inhalation précédente pour qu'elle soit une durée moyenne de trois inhalations précédentes.

7. Dispositif d'administration de gaz thérapeutique selon la revendication 1 dans lequel le dispositif de commande est en outre configuré pour, après l'arrêt, fournir une précharge de gaz thérapeutique à la fois aux premier et second raccords de tuyau avant une inhalation ultérieure.

8. Dispositif d'administration de gaz thérapeutique selon la revendication 1 dans lequel le dispositif de commande est en outre configuré pour, après l'arrêt, fournir une précharge de gaz thérapeutique à au moins l'un des premier et second raccords de tuyau avant une inhalation ultérieure.

9. Dispositif d'administration de gaz thérapeutique selon la revendication 8, dans lequel chacun des premier et second raccords de tuyau définit un volume luminal, et dans lequel lorsque le dispositif de commande remplit l'au moins un des premier et second raccords de tuyau, le dispositif de commande est configuré pour fournir à l'au moins un des premier et second raccords de tuyau un volume de remplissage qui est à plus ou moins dix pour cent (+/-10 %) du volume luminal.

10. Dispositif d'administration de gaz thérapeutique selon la revendication 1 dans lequel le dispositif de commande est en outre configuré pour déterminer, sur la base des premier et second capteurs, qu'un volume de flux plus important s'écoule à travers le premier raccord de tuyau.

11. Système comprenant :
une source de gaz thérapeutique ;
une canule nasale bifurquée ;
un dispositif d'administration de gaz thérapeutique selon une quelconque revendication précédente, dans lequel le dispositif d'administration de gaz thérapeutique est accouplé à la source de gaz thérapeutique, et accouplé à la canule nasale bifurquée.

12. Système selon la revendication 11 dans lequel la source de gaz thérapeutique est un concentrateur d'oxygène.
